# EUROPÄISCHE PATENTSCHRIFT

(11) **EP 0 981 764 B1**
(45) Veröffentlichungstag und Bekanntmachung des Hinweises auf die Patenterteilung: **19.07.2006**
(21) Anmeldenummer: 98930682.4
(22) Anmeldetag: 12.05.1998
(51) Int. Cl.: G01S 15/89, B06B 1/06

(54) **MULTIFREQUENZ-ULTRASCHALLSONDE**
MULTIFREQUENCY ULTRASOUND PROBE
SONDE A ULTRASONS MULTIFREQUENCE

(30) Priorität: 12.05.1997 DE 29708338 U
(43) Veröffentlichungstag der Anmeldung: 01.03.2000
(73) Patentinhaber: Compumedics Germany GmbH, 78224 Singen (DE)
(72) Erfinder: BRUCHER, Rainer, D-89173 Lonsee (DE); WIEST, Achim, D-88662 Überlingen (DE); WIDENHORN, Gerold, D-88662 Überlingen (DE)
(74) Vertreter: Hiebsch, Gerhard F.
(86) Internationale Anmeldenummer: PCT/EP1998/002771
(87) Internationale Veröffentlichungsnummer: WO 1998/052068

(56) Entgegenhaltungen:
- DE-A- 3 008 553
- DE-A- 3 441 563
- GB-A- 1 266 143
- US-A- 2 645 727
- US-A- 4 459 853
- US-A- 4 841 977
- US-A- 4 872 346
- US-A- 5 348 015
- US-A- 5 410 205

## Beschreibung

Die vorliegende Erfindung betrifft eine Ultraschallsonde nach dem Oberbegriff des Patentanspruchs 1.

In der medizinischen Diagnostik, insbesondere der Ultraschall- Sonographie, gibt es zahlreiche Anwendungsmöglichkeiten für derartige Vorrichtungen. So werden Ultraschallsonden insbesondere im Zusammenhang mit medizinischen Ultraschallvorrichtungen verwendet, die auf dem Dopplerprinzip beruhen, und die für einen jeweiligen diagnostischen Zweck geeignete Ultraschallsignale im Bereich zwischen etwa einem und etwa 20 MHz erzeugen. In entsprechender Weise sind auch die Ultraschallsonden an derartige (Einzel-) Frequenzen angepaßt.

Aus dem Stand der Technik bekannte Ultraschallsonden bestehen üblicherweise aus einem Piezokristall, welcher in geeigneter Weise elektrisch erregt wird, um dann ein Ultraschallsignal, etwa 2,4, 8 oder 16 MHz, abzugeben, wobei der Kristall für eine jeweilige Frequenz bestimmte, geometrische und elektrische Parameter besitzt.

Insbesondere auf dem technischen Gebiet der Embolusdetektion mittels Ultraschall, einem neuen und medizinisch bedeutsamen Anwendungsgebiet der transkraniellen Doppler-Sonographie, stellt sich die technische Herausforderung, in einem mittels Ultraschall überwachten Blutgefäß zuverlässig und unterscheidbar das Auftreten von Embolien zu erkennen, wobei ein Embolus sich durch -- gegenüber dem umgebenden Blut -- charakteristische Reflexionseigenschaften des einfallenden Ultraschallsignals auszeichnet. Allerdings ist nach wie vor die Unterscheidung zwischen eigentlichen Embolien und (unerwünschten) Störeffekten, etwa durch Sondenbewegung entstehenden Artefakten, schwierig.

Es hat sich herausgestellt, daß insbesondere Embolien sich zusätzlich durch charakteristische, frequenzabhängige Reflexionseigenschaften eines oder mehrerer einfallender Ultraschallsignale erkennen und unterscheiden lassen, wobei etwa das reflektierte Signal auf einer ersten Ultraschallfrequenz deutlich höher oder niedriger sein kann, als der entsprechende, reflektierte Signalpegel bei einer zweiten Frequenz.

DE 30 08 553 A1 offenbart eine Ultraschallsonde gemäß dem Oberbegriff von Anspruch 1.

Aufgabe der vorliegenden Erfindung ist es daher, eine verbesserte Ultraschallsonde zu schaffen, die für einen einfachen und bedienungsfreundlichen Multifrequenzbetrieb geeignet ist, also mehr als eine Ultraschallfrequenz (bzw. ein zusammenhängendes Ultraschallband) gleichzeitig auf einen Fokusspunkt im Beobachtungsmedium abstrahlen bzw. das davon reflektierte Signal aufnehmen kann.

Die Aufgabe wird durch die Ultraschallsonde nach Anspruch 1 gelöst.

Vorteilhaft wird dabei -- bedingt durch die strukturelle bzw. geometrische Einheit des beteiligten Einzelkristalls -- stets eine optimale, deckungsgleiche Fokussierung der gleichzeitig abgestrahlten, schmalbandigen Trägersignale erreicht, und insbesondere Einstellungsarbeiten bzw. Justierungen durch eine Bedienperson sind nicht mehr notwendig.

Auch läßt sich auf die erfüllungsgemäße Weise eine überaus kompakte Sonde herstellen, die sowohl für die Fertigung als auch für den praktischen Betrieb deutliche Vorteile hinsichtlich Trageverhalten und Bedienung zeigt.

In ansonsten bekannter Weise wird dann das empfangene, reflektierte Dopplersignal -- bevorzugt mehrkanaligeiner Auswertung zugeführt, die dann insbesondere auch zur frequenz- (muster-) abhängigen Emboliedetektion eingerichtet sein kann.

Dabei ist im Grundsatz die Ultraschallsonde gemäß der vorliegenden Erfindung für beliebige, zu überwachende Medien -- keinesfalls beschränkt auf Blutgefäße -- geeignet.

Wesentliches Merkmal der Erfindung ist auch, daß das Mehrfrequenzsignal für den Ultraschallgeber ein simultanes und synchronisiertes Mehrfrequenzsignal ist, wobei bevorzugt die einzelnen Trägerfrequenzen zueinander in einem nicht-harmonischen Verhältnis stehen.

Vorteilhafte Weiterbildungen der Erfindung sind in den Unteransprüchen beschrieben.

Sowohl für diese Ausführungsform als auch für die weiteren Realisierungsformen der Erfindung gelten sowohl die Serien- als auch die Parallelresonanz als Resonanzfrequenz im Sinne der Erfindung.

Weitere Vorteile, Merkmale und Einzelheiten der Erfindung ergeben sich aus der Beschreibung, sowie aus der anliegenden Figur.

Diese zeigt:

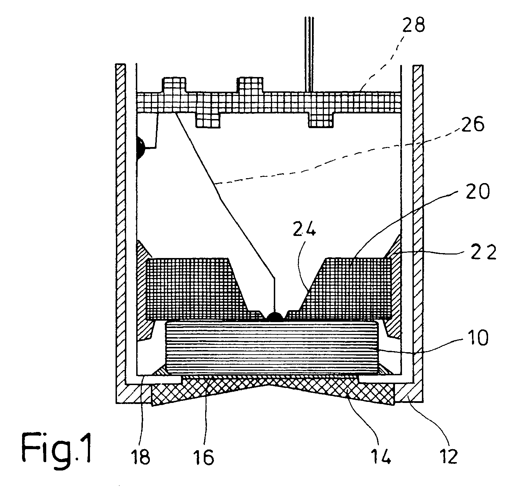

- Fig.1:: eine seitliche Schnittansicht einer erfindungsgemäßen Ultraschallsonde gemäß einer ersten, bevorzugten Ausführungsform der Erfindung;

Fig. 1 zeigt die Ausführungsform der erfindungsgemäßen Ultraschallsonde. Ein Piezokristall 10, dessen Resonanzfrequenzen (Serie- und Parallelresonanzfrequenz) auf dem Bereich zwischen 2 und 2,5 MHz eingerichtet ist, bedeckt die bodenseitige Öffnung eines becherförmigen Kunststoffgehäuses 6. Dessen bodenseitige Öffnung ist zusätzlich durch ein -- entsprechend dem zylindrischen Kristall 10 -- kreisförmiges Linsenelement 14 aus geeignetem Kunststoffmaterial abgeschlossen, welches zur -- fokussierenden oder nicht-fokussierenden -- Koppelung der Ultraschallschwingungen des Piezokristalls 10 in einen zu überwachenden Körper dienen soll. Hierbei ist anzustreben, daß die Foci beider Frequenzen für die Emboliedetektion möglichst deckungsgleich sind, insbesondere betreffend die Intensitätsverteilung.

Zwischen dem Kristall 10 und der Linse 14 ist eine dünne Klebeschicht 16, bevorzugt aus Silberleitkleber einer Dicke im Bereich einiger Mikrometer, zur Ankoppelung vorgesehen.

Darüber hinaus ist im Innenbereich des Gehäuses 12 diese von einem schirmenden Innengehäuse 18, bevorzugt aus Kupfermaterial, ausgekleidet. An diesem Abschirmmantel 18 ist seitlich nicht nur der Piezokristall 10 befestigt, sondern auch ein auf dem Kristall der Linse 14 gegenüberliegend vorgesehenes Anpaßelement ("Backing") 20 aus Metall oder Glas, welches mittels einer radial umlaufenden Klebung 22 an dem Mantel 18 befestigt ist. Das Anpaßelement 20 weist eine i.w. zentrische Bohrung 24 auf, die im gezeigten Ausführungsbeispiel konisch ist, einen mittigen Durchbruch zur Oberfläche des Kristalls 10 besitzt und entsprechend der gewünschten Resonanzfrequenz bzw. der Breitbandigkeit (Güte) des Piezokristalls 10 bemessen ist. Durch diesen zentralen Durchbruch greift zudem eine Signalleitung 26, die elektrisch den Kristall 10 mit einer Verstärkereinheit 28, symbolisiert durch eine Leiterplatte, verbindet.

Dieser Verstärker 28 ist im dargestellten Ausführungsbeispiel als zweikanalige Verstärkereinheit mit zwei parallelen Kanälen ausgebildet, um die jeweiligen Einzelfrequenzen -- 2 MHz sowie 2,5 MHz -- aufbereiten und zur weiteren Auswertung weiterleiten zu können.

Die konkreten Abmessungen von Kristall 10, Backing 20 sowie dessen Materialwahl -- bevorzugt Metall oder Glassind abhängig von den jeweils gewünschten Resonanzfrequenzen bzw. einer beabsichtigen Bandbreite, die breit genug sein muß, um die -- synchronisierten -- Sendefrequenzen in der gewünschten Weise abstrahlen zu können.

Erfindungsgemäß wird also durch diese Ausführungsform bewußt die Güte eines Ultraschall-Piezokristalls verringert, womit dessen Bandbreite (für die Multifrequenz) erhöht wird. Technisch wird dies durch die besonders zugeordnete, rückwärtige Anpassung (Backing) erreicht, womit trotz dieser Einkristallsonde dann diese multifrequenzfähig wird.

Mit der dargestellten Ausführungsform ist somit das Senden und Empfangen von zwei verschiedenen Zentralfrequenzen (hier beispielhaft: 2 MHz und 2,5 MHz) mittels einer einzigen Sonde möglich, die dann insbesondere auch bevorzugt zur frequenzdiskriminierenden Analyse von an Embolien oder anderen Körpern in Blutfluß reflektierten Doppler-Ultraschallsignalen vorteilhaft benutzt werden kann.

## Patentansprüche

1. Ultraschallsonde, insbesondere für Anwendungen im Bereich der medizinischen Diagnostik mittels Ultraschallsonographie, mit einem zum Erzeugen eines Ultraschall-Sendesignals als Reaktion auf eine elektrische Erregung und zum Empfangen eines an einem Beobachtungsmedium reflektierten Empfangssignals ausgebildeten Ultraschallgeber (10;10ₐ,10_{b};A,B), der in einem Gehäuse (12) gehalten und mit Koppel- bzw. Übertragungsmitteln (14) zum Einkoppeln des Sendesignals in das Beobachtungsmedium versehen ist, wobei das Sendesignal ein Mehrfrequenzsignal, bestehend aus mindestens zwei, im Frequenzbereich voneinander beabstandeten Einzelsignalen ist, und Mittel zum Auswerten des reflektierten Empfangssignals abhängig von den Einzelsignalen vorgesehen sind, wobei der Ultraschallgeber einen Einzelkristall (10) zur Signalerzeugung aufweist, welcher mit dem Mehrfrequenzsignal mit mehreren Trägerfrequenzen in der Nähe seiner Resonanzfrequenz simultan und synchronisiert beaufschlagt wird und auf einer rückwärtigen, dem Beobachtungsmedium abgewandten Fläche mit einem im Innenbereich des Gehäuses vorgesehenen Anpassungselement versehen ist, **dadurch gekennzeichnet, daß** das Anpassungselement (20) aus Metall oder Glas besteht und zur vorbestimmbaren Beeinflussung der Bandbreite und/oder der Resonanzfrequenz einen mittigen Durchbruch zur Oberfläche des Einzelkristalls (10) besitzt.

2. Ultraschallsonde nach Anspruch 1, **dadurch gekennzeichnet, dass** die als Linsenelement (14) ausgebildeten Koppel- bzw. Übertragungsmittel eine bodenseitige Öffnung des Gehäuses verschließen.

3. Ultraschallsonde nach Anspruch 2, **dadurch gekennzeichnet, dass** zwischen dem Ultraschallgeber und dem Linsenelement eine Klebeschicht zur Ankoppelung vorgesehen ist.

4. Ultraschallsonde nach einem der Ansprüche 1 bis 3, **dadurch gekennzeichnet, dass** durch den Durchbruch eine elektrisch den Einzelkristall (10) mit einer Verstärkereinheit (28) verbindende Signalleitung (26) geführt ist.

5. Ultraschallsonde nach einem der Ansprüche 1 bis 4, **dadurch gekennzeichnet, dass** das Mehrfrequenzsignal die Trägerfrequenzen 2 und 2,5 MHz oder die Trägerfrequenzen 1,7 und 2, 4 MHz aufweist.

6. Verwendung der Ultraschallsonde nach einem der Ansprüche 1 bis 5 zur frequenzdiskriminierenden Analyse von an Embolien oder anderen Körpern in einem Blutfluss reflektierten Doppler-Ultraschallsignalen.

## Claims

1. Ultrasonic probe, in particular for applications in the field of medical diagnosis by ultrasound sonography, with an ultrasonic generator (10; 10ₐ, 10_{b}; A, B) which is designed to generate an ultrasound transmission signal as a reaction to electrical excitation and to receive a reception signal reflected on an observation medium and which is held in a housing (12) and provided with coupling or transmission means (14) for input of the transmission signal into the observation medium, wherein the transmission signal comprises a multi-frequency signal consisting of two or more individual signals spaced apart from each other in the frequency range, and means for evaluating the reflected reception signal as a function of the individual signals, wherein the ultrasonic generator comprises a single crystal (10) for signal generation to which with the multi-frequency signal several carrier frequencies in the vicinity of its resonant frequency are applied simultaneously and synchronously and which on a rear surface facing away from the observation medium is provided with an adapter element (20) provided in the inner region of the housing, **characterised in that** the adapter element (20) is made of metal or glass and has a central aperture to the surface of the single crystal (10) for presettable control of the bandwidth and/or resonant frequency.

2. Ultrasonic probe according to claim 1, **characterised in that** the coupling or transmission means designed as a lens element (14) close an opening in the bottom of the housing.

3. Ultrasonic probe according to claim 2, **characterised in that** between the ultrasonic generator and the lens element is provided an adhesive layer for coupling.

4. Ultrasonic probe according to any of claims 1 to 3, **characterised in that** through the aperture passes a signal wire (26) electrically connecting the single crystal (10) to an amplifier unit (28).

5. Ultrasonic probe according to any of claims 1 to 4, **characterised in that** the multi-frequency signal exhibits the carrier frequencies 2 and 2.5 MHz or the carrier frequencies 1.7 and 2.4 MHz.

6. Use of the ultrasonic probe according to any of claims 1 to 5 for frequency-discriminating analysis of Doppler ultrasound signals reflected on embolisms or other bodies in a blood flow.

## Revendications

1. Sonde à ultrasons, en particulier pour des applications dans le domaine du diagnostic médical, par sonographie à ultrasons avec un émetteur d'ultrasons (10 ;10ₐ, 10_{b} ; A, B), qui est réalisé pour produire un signal d'émission d'ultrasons en réaction à une excitation électrique et pour recevoir un signal de réception réfléchi sur un milieu d'observation, qui est maintenu dans un boîtier (12) et muni de moyens de couplage et/ou de transmission (14) pour le couplage du signal d'émission dans le milieu d'observation, le signal d'émission étant un signal à plusieurs fréquences composé d'au moins deux signaux individuels, espacés l'un de l'autre dans la plage des fréquences, et de moyens d'évaluation du signal de réception réfléchi en fonction des signaux individuels étant prévus, l'émetteur d'ultrasons comportant un monocristal (10) pour la production de signaux, qui est sollicité, simultanément et de façon synchronisée, par le signal à plusieurs fréquences, avec plusieurs fréquences porteuses à proximité de sa fréquence de résonance, et est muni, sur une face arrière, opposée au milieu d'observation, d'un élément d'adaptation, prévu dans la zone intérieure du boîtier **caractérisé en ce que** l'élément d'adaptation (20) est composé de métal ou de verre et comprend un passage, central par rapport à la surface du monocristal (10), pour influer de façon prédéterminable sur la largeur de bande et/ou sur la fréquence de résonance.

2. Sonde à ultrasons selon la revendication 1, **caractérisée en ce que** les moyens de couplage et/ou de transmission, réalisées sous la forme d'éléments lenticulaires (14), obturent une ouverture du boîtier du côté du fond.

3. Sonde à ultrasons selon la revendication 2, **caractérisée en ce qu'**une couche adhésive est prévue entre l'émetteur d'ultrasons et l'élément lenticulaire, pour assurer le couplage.

4. Sonde à ultrasons selon l'une des revendications 1 à 3, **caractérisée en ce qu'**une ligne de signalisation (26), reliant électriquement le monocristal (10) à une unité amplificatrice (28), est guidée à travers le passage.

5. Sonde à ultrasons selon l'une des revendications 1 à 4, **caractérisée en ce que** le signal à plusieurs fréquences présente les fréquences porteuses de 2 et 2,5 Mhz ou les fréquences porteuses de 1,7 et 2,4 Mhz.

6. Utilisation de la sonde à ultrasons selon l'une des revendications 1 à 5, pour l'analyse à discrimination fréquentielle de signaux à ultrasons Doppler, réfléchis sur des embolies ou sur d'autres corps dans un flux sanguin.
